# EUROPÄISCHE PATENTANMELDUNG

(11) **EP 1 035 195 A1**
(43) Veröffentlichungstag der Anmeldung: **13.09.2000**
(21) Anmeldenummer: 99121586.4
(22) Anmeldetag: 29.10.1999
(51) Int. Cl.: C11D 3/50, C11D 1/88, C11D 1/94, A61L 9/04

(54) **Zubereitung, insbesondere für WC-Erfrischer**

(30) Priorität: 11.03.1999 DE 19910878; 12.05.1999 DE 19922040
(71) Anmelder: drom fragrances international KG, 82065 Baierbrunn (DE)
(72) Erfinder: Meller, Gerhard, 53879 Euskirchen (DE)
(74) Vertreter: Patentanwälte Dr. Solf & Zapf

(57) **Zusammenfassung**

Eine Zubereitung, insbesondere für WC-Erfrischer, umfassend Hilfs- und Duftstoffe sowie Tenside und ist dadurch gekennzeichnet, daß als Tensid wenigstens ein im neutralen bis sauren Bereich stabiles amphoteres Tensid enthalten ist, wodurch sich ein klares, mäßig schäumendes Produkt ergibt.

## Beschreibung

Die Erfindung betrifft eine Zubereitung, insbesondere für WC-Erfrischer.

Bekannte WC-Erfrischer umfassen beispielsweise WC-Steine, die in Körbchen unter den Beckenrand gehängt bzw. direkt in das Spülbecken eingebracht werden, um beim Spülvorgang die Inhaltsstoffe der WC-Erfrischer in das Becken der Toilette zu spülen. Andere bekannte WC-Erfrischer bestehen aus Gelen oder umfassen Einhängesysteme, die mit einem Nachfüllbehältnis für eine flüssige Zubereitung versehen sind.

Die bekannten Zubereitungen für WC-Erfrischer können Hilfs- und Duftstoffe sowie antibakterielle Mittel ebenso wie Mittel zur Verhinderung von Kalkablagerungen enthalten.

Aus der EP 0 775 741 A1 sind flüssige Zubereitungen für WC-Erfrischer bekannt, welche anionische und/oder nichtionische Tenside enthalten.

Die Verwendung anionischer Zubereitungen ist jedoch einerseits mit dem Nachteil verbunden, daß je nach Typ der Toilette beim Spülvorgang eine hohe Schaumentwicklung entstehen kann und des weiteren dürfen solche anionischen Zubereitungen keine kationischen Tenside als bakterizide und fungizide Mittel enthalten. Bei Zubereitungen, die aus nichtionischen Tensiden bzw. aus einer Mischung aus nichtionischen und anionischen Tensiden bestehen, tritt weiterhin ein Problem der Lagerbeständigkeit auf, da eine längere Lagerzeit zu einem Viskositätsanstieg führen kann, wodurch sich Dosierprobleme und auch Schaumprobleme ergeben können.

Der Erfindung liegt daher die Aufgabe zugrunde, eine klare Zubereitung, insbesondere für WC-Erfrischer zu schaffen, deren Viskosität auch bei längerer Lagerung weitgehend unverändert bleibt und bei deren Anwendung sich keine größeren Mengen stabiler Schäume bilden und die in der Lage ist größere Mengen Parfümöl aufzunehmen.

Diese Aufgabe wird durch die im Anspruch 1 angegebene Zubereitung gelöst. Die Unteransprüche 1 bis 17 geben vorteilhafte Ausgestaltungen der Zubereitung nach Anspruch 1 an.

Die Ansprüche 18 bis 21 beschreiben eine besonders wirkungsvolle und günstige Zubereitung für WC-Erfrischer.

Gegenstand der Erfindung ist daher eine Zubereitung, insbesondere für WC-Erfrischer, die neben an sich bekannten Hilfs- und Duftstoffen wenigstens ein im sauren bis neutralen Bereich stabiles amphoteres Tensid enthält.

Besonders günstige Ergebnisse liefern amphotere Tenside vom Betain-Typ, ausgewählt aus:
Kokosalkyldimethylammoniumbetain, Kokosamidopropylbetain, Lauryldimethylhydroxysulfobetain, Lauryl- und Myristylbetain, Alkyldimethylbetain, Lauryldimethylaminoessigsäurebetain und Alkylamidopropylbetain, wobei die Alkylgruppen linear oder verzweigt sein können und 8 bis 22 Kohlenstoffatome, vorzugsweise 12 bis 16 Kohlenstoffatome aufweisen.

Bei einer bevorzugten Ausführungsform kann die Zubereitung auch Mischungen verschiedener amphoterer Tenside vom Betain-Typ enthalten. Für den Fall, daß die erfindungsgemäße Zubereitung, insbesondere zur Verhinderung von Kalkablagerungen im Toilettenbecken, auf einen sauren Bereich eingestellt wird, werden bevorzugt solche Betaine verwendet, die keinen Amidrest im Molekül aufweisen, wie Kokosalkyldimethylammoniumbetain, wobei die Alkylgruppe linear oder verzweigt sein kann und 8 bis 22 Kohlenstoffatome, vorzugsweise 12 bis 16 Kohlenstoffatome aufweist.

Ebenso vorteilhafte Ergebnisse liefern amphotere Tenside vom Aminoxid-Typ, die ausgewählt sind aus Cocodimethylaminoxid und Decyldimethylaminoxid.

Überraschenderweise wurde gefunden, daß die erfindungsgemäßen Zubereitungen, insbesondere für WC-Erfrischer auch im niedrigen pH-Bereich von z.B. pH 3 bis pH 7 zu klaren Lösungen führen, und beim Spülvorgang keine hohe Schaumentwicklung bzw. Bildung stabiler Schäume auftritt.

Diese erfindungsgemäßen Zubereitungen sind bei etwa 3 bis 50 °C lagerstabil mit gutem Netz- und Dispergierverhalten und stabiler Viskosität bei 5 bis 40 °C, so daß sie bei ihrer Anwendung eine gute Reinigungswirkung entfalten.

Zur Geruchsverbesserung können die erfindungsgemäßen Zubereitungen beispielsweise Parfümöle enthalten, wobei sich als zusätzliche Emulgatoren für die lipophilen Bestandteile und zum Aufbau eines Teiles der Strukturviskosität nichtionische Tenside vorteilhaft einsetzen lassen. Durch die Verwendung nichtionischer Tenside läßt sich das Anschäumverhalten verbessern, ohne daß sich ein unerwünschter stabiler Schaum aufbaut.

Als nichtionische Tenside werden vorzugsweise Fettsäureglyceride, Sorbitanester oder auch Fettalkoholethoxylate basierend auf Fettsäuren, Fettalkoholen oder Fettsäurederivaten mit 11 bis 18 Kohlenstoffatomen und einem Oxethylierungsgrad von 2-20 Mol EO, ebenso wie Fettsäurealkanolamide auf Basis von Kokosfettsäure mit Diethanolamin verwendet werden.

Bevorzugt werden Polyolfettsäureester eingesetzt, welche aus Polyethylenglycol und Glyceryloleat aufgebaut sind, wobei das Polyethylenglycol 2 bis 150 Kohlenstoffatome, vorzugsweise 14 bis 22 Kohlenstoffatome und das Glyceryloleat 8 bis 22, vorzugsweise 12 bis 16 Kohlenstoffatome aufweist.

Insbesondere wird als nichtionisches Tensid der erfindungsgemäßen Zubereitung Isotridecylalkoholpolyglykolether, vorzugsweise mit 5-8 Mol EO zugesetzt, wodurch sich eine gewünschte Strukturviskosität ausbildet und die Schaumbildung auf ein gewünschtes Maß eingestellt werden kann. Die gemeinsame Verwendung eines amphoteren Tensids vom Betain-Typ mit Isotridecylalkoholpolyglykolether als nichtionischem Tensid führt überraschenderweise zu dem Ergebnis, daß sich dadurch große Parfummengen klar lösen lassen ohne daß die übrigen Produkteigenschaften der flüssigen Zubereitung beeinflußt wird.

Beispiele für in der Erfindung verwendbare nichtionische weitere Tenside sind Sorbitanmonolaurat, Sorbitansesquioleat, Fettalkohol C12 bis C14, Polyoxyethylen-24-glycerinmonostearat, Kokosfettsäurediethanolamid und Isotridecylalkoholpolyglykolether mit 5 bis 8 Mol EO, und Dinatriumlaurethsulfosuccinat.

Die nichtionischen Tenside sind in einer Menge von 1 bis 10 Gew.-%, vorzugsweise 3 bis 7 Gew.-% in der Zubereitung enthalten.

Das amphotere Tensid vom Betain-Typ bzw. vom Aminoxid-Typ ist in der Zubereitung in einer Menge von 5 bis 40 Gew.-%, vorzugsweise 5 bis 30 Gew.-%, insbesondere 5 bis 20 Gew.-% enthalten.

Bei einer weiteren bevorzugten Ausführungsform enthält die erfindungsgemäße Zubereitung Mittel mit bakteriziden und fungiziden Eigenschaften, wie kationische Tenside z.B. quarternäre Ammoniumverbindungen oder Amine bzw. Diamine. Diese Verbindungen entfalten je nach Typ ihre Wirksamkeit schon bei sehr geringen Einsatzmengen bis zu 5 Gew.-%.

Überraschenderweise wurde festgestellt, daß die kationischen Tenside vom vorgenannten Typ mit den amphoteren Tensiden vom Betain-Typ bzw. Aminoxid-Typ kompatibel sind und sich daher die hohen bakteriziden und fungiziden Eigenschaften der quarternären Ammoniumverbindungen bzw. Amine oder Diamine bei der Herstellung der erfindungsgemäßen Zubereitungen nutzen lassen, so daß damit WC-Erfrischer herstellbar sind, die bakteriostatische bis desinfizierende Wirkungen aufweisen.

Bevorzugte quaternäre Ammoniumverbindungen sind Alkyldimethylbenzylammoniumchlorid mit einer linearen oder verzweigten Alkylgruppe mit 8 bis 22 Kohlenstoffatomen, vorzugsweise 12 bis 18 Kohlenstoffatomen. Beispiele hierfür sind Diedecyldimethylammoniumchlorid, Octyldecyldimethylammoniumchlorid, Dioctyldimethylammoniumchlorid, Didecyldimethylammoniumchlorid.

Im folgenden wird die Erfindung anhand von Beispielen erläutert, wobei sich die angegebenen Prozentzahlen auf das Gewicht beziehen, wobei der Fachmann weiß, daß diese Rezeptur im Rahmen des fachmännischen Könnens modifiziert werden kann.

| Beispiel 1: | |
|---|---|
| Dehyton AB 30 r | 5 - 40 Gew.-% |
| Genapol X-080 r | 1 - 10 Gew.-% |
| Zitronensäure | 1 - 6 Gew.-%Zitronensäure |
| | 1 - 6 Gew.-% |
| Dipropylenglykol | 1 - 10 Gew.-% |
| Rhodopol T r | 0,1 - 0,5 Gew.-% |
| Parfümöl DROM r | 2 - 15 Gew.-% |
| Konservierungsmittel | q.s. |
| Farbe | q.s. |
| Wasser | ad 100 Gew.-% |

Dehyton AB 30 r: Kokosalkyldimethylammoniumbetain (30%), Genapol X-080 r: Isotridecylalkoholpolyglykolether 8 EO und Rhodopol T r: Xanthan.

| Beispiel 2: | |
|---|---|
| Lauryldimethylaminoessigsäurebetain | 5 - 30 Gew.-% |
| Isotridecylalkoholpolyglykolether 8 Mol/EO | 1 - 10 Gew.-% |
| Zitronensäure | 1 - 6 Gew.-% |
| Dipropylenglykol | 1 - 10 Gew.-% |
| Polyolfettsäureester | 0,5 - 6 Gew.-% |
| (solche gemäß Beschreibung) Parfümöl | 2 - 15 Gew.-% |
| Konservierungsmittel | q.s. |
| Farbe | q.s |
| Wasser | ad 100 Gew.-% |

| Beispiel 3: | |
|---|---|
| Cocodimethylaminoxid | 5 - 30 Gew.-% |
| Isotridecylalkohlpolyglykolether 8 Mol/EO | 1 - 10 Gew.-% |
| Zitronensäure | 1 - 6 Gew.-% |
| Dipropylenglykol | 1 -10 Gew.-% |
| Polyolfettsäureester | 0,5 - 6 Gew.-% |
| (solche gemäß Beschreibung) Parfümöl | 2 - 15 Gew.-% |
| Konservierungsmittel | q.s. |
| Farbe | q.s. |
| Wasser | ad 100 Gew.-% |

| Beispiel 4 | |
|---|---|
| Alkylamidopropylbetain | 1 - 50 Gew.-% |
| (solche gemäß Beschreibung) Fettaminpolyethylenglykolether C₆-C₂₂, 1-20 Mol EO | 1 - 10 Gew.-% |
| Polyolfettsäureester | 0 - 5 Gew.-% |
| (solche gemäß Beschreibung) Dinatriumlaurethsulfosuccinat | 0 - 5 Gew.-% |
| Parfümöl | 1 - 15 Gew.-% |
| Farbe | q.s. |
| Konservierungsmittel | q.s. |
| Zitronensäure | q.s. |
| Wasser | ad 100 Gew.-% |

Die erfindungsgemäße Zubereitung nach Beispiel 1 wird wie folgt hergestellt:
Zunächst werden Dehyton, Genapol und Dipropylenglykol und Wasser gemischt und vorgemischtes Parfümöl und Rhodopol werden unter Rühren zu dem Tensid-Wassergemisch zugegeben. Dabei wird kalt so lange gerührt, bis sich das Rhodopol vollständig gelöst hat. Abschließend werden zur Einstellung des pH-Wertes Zitronensäure sowie Konservierungsmittel und Farbe nach Belieben zugegeben.

Die Zubereitungen gemäß Beispielen 2 und 3 werden analog hergestellt.

Zur Herstellung der Zubereitung gemäß Beispiel 4 wird das Parfümöl mit dem Alkylamidopropylbetain, dem Fettaminopolyethylenglykolether, dem Polyolfettsäureester und dem Dinatriumlaurylsulfosuccinat gemischt und in Wasser eingerührt. Anschließend wird je nach Bedarf Konservierungsmittel und Farbe zugegeben und gegebenenfalls der pH-Wert eingestellt. Bei der Herstellung dieser Zubereitung soll ein unnötiger Lufteinzug vermieden werden. Für die Herstellung gemäß Beispiel 4 können auch sämtliche Rohstoffe, beispielsweise in ein Becherglas, eingewogen werden und werden anschließend solange gerührt, bis eine klare Lösung entsteht, wonach gegebenenfalls wiederum der pH-Wert eingestellt wird.

Die erfindungsgemäße flüssige Zubereitung läßt sich in beliebige Dosiersysteme einfüllen und läßt sich vielseitig beispielsweise zur Lufterfrischung bis hin zum antimikrobiellen WC-Erfrischer bzw- Reiniger verwenden.

## Patentansprüche

1. Zubereitung, insbesondere für WC-Erfrischer, umfassend Hilfs- und Duftstoffe sowie Tenside,
**dadurch gekennzeichnet,**
daß die Zubereitung wenigstens ein im neutralen bis sauren Bereich stabiles amphoteres Tensid enthält.

2. Zubereitung nach Anspruch 1,
**dadurch gekennzeichnet,**
daß das amphotere Tensid vom Betain-Typ ist.

3. Zubereitung nach Anspruch 1 oder 2,
**dadurch gekennzeichnet,**
daß das amphotere Tensid vom Betain-Typ ausgewählt ist aus Kokosalkyldimethylammoniumbetain, Kokosamidopropylbetain, Lauryldimethylhydroxysulfobetain, Lauryl- und Myristylbetain, Alkyldimethylbetain, Lauryldimethylaminoessigsäurebetain und Alkylaminopropylbetain, wobei die Alkylgruppen linear oder verzweigt sein können und 8 bis 22 Kohlenstoffatome, vorzugsweise 12 bis 16 Kohlenstoffatome aufweisen.

4. Zubereitung nach Anspruch 2,
**dadurch gekennzeichnet,** daß
das amphotere Tensid vom Betain-Typ keinen Amidrest im Molekül aufweist.

5. Zubereitung nach Anspruch 4,
**dadurch gekennzeichnet,**
daß das amphotere Tensid, Kokosalkyldimethylammoniumbetain ist.

6. Zubereitung nach Anspruch 1,
**dadurch gekennzeichnet,**
daß das amphotere Tensid vom Aminoxid-Typ ist.

7. Zubereitung nach Anspruch 6,
**dadurch gekennzeichnet,**
daß das amphotere Tensid vom Aminoxid-Typ ausgewählt ist aus Cocodimethylaminoxid und Decyldimethylaminoxid.

8. Zubereitung nach einem der Ansprüche 1 bis 7,
**dadurch gekennzeichnet,**
daß die Zubereitung wenigstens ein nichtionisches Tensid enthält.

9. Zubereitung nach Anspruch 8,
**dadurch gekennzeichnet,**
daß das nichtionische Tensid ausgewählt ist aus Fettsäureglyceriden, Sorbitanestern und Fettalkoholethoxylaten basiernd auf Fettsäuren, Fettalkoholen oder Fettsäurederivaten mit 11 bis 18 Kohlenstoffatomen und einem Oxethylierungsgrad von 2 bis 20 Mol EO, sowie aus Fettsäurealkanolamiden auf Basis von Kokosfettsäure mit Diethanolamin, sowie Polyolfettsäureester, welche aus Polyethylenglycol und Glyceryloleat aufgebaut sind, wobei das Polyethylenglycol 2 bis 150 Kohlenstoffatomen, vorzugsweise 14 bis 22 Kohlenstoffatome und das Glyceryloleat 8 bis 22, vorzugsweise 12 bis 16 Kohlenstoffatome aufweist.

10. Zubereitung nach Anspruch 9,
**dadurch gekennzeichnet,**
daß das nichtionische Tensid Sorbitanmonolaurat, Sorbitansesquioleat, Fettalkohol C12 bis C14, Polyoxyethylen-24-glycerinmonostearat, Kokosfettsäurediethanolamid und Isotridecylalkoholpolyglykolether mit 5 bis 8 Mol EO, und Dinatriumlaurethsulfosuccinat, ist.

11. Zubereitung nach einem der Ansprüche 1 bis 10,
**dadurch gekennzeichnet,**
daß sie wenigstens eine quarternäre Ammoniumverbindung ausgewählt aus Alkyldimethylbenzylammoniumchloriden, wobei die Alkylgruppe linear oder verzweigt sein kann und 8 bis 12 Kohlenstoffatome, vorzugsweise 12 bis 18 Kohlenstoffatome aufweist, ausgewählt aus Didecyldimethylammoniumchlorid, Octyldecyldimethylammoniumchlorid, Dioctyldimethylammoniumchlorid, Didecyldimethylammoniumchlorid.

12. Zubereitung nach einem der Ansprüche 1 bis 11,
**dadurch gekennzeichnet,**
daß die Zubereitung auf einen pH-Wert zwischen 2 bis 6 eingestellt ist.

13. Zubereitung nach Anspruch 12,
**dadurch gekennzeichnet,**
daß sie auf einen pH-Wert von 3 bis 4 eingestellt ist.

14. Zubereitung nach einem der Ansprüche 1 bis 13,
**dadurch gekennzeichnet,**
daß sie Zitronensäure enthält.

15. Zubereitung nach einem der Ansprüche 1 bis 13,
**dadurch gekennzeichnet,**
daß sie Ameisensäure enthält.

16. Zubereitung nach einem der Ansprüche 1 bis 14,
**dadurch gekennzeichnet,**
daß das amphotere Tensid in einer Menge von 5 bis 40 Gew.-% enthalten ist.

17. Zubereitung nach einem der Ansprüche 1 bis 15,
**dadurch gekennzeichnet,**
daß sie Parfümöl enthält.

18. Zubereitung, insbesondere für WC-Erfrischer umfassend:
| | |
|---|---|
| Dehyton AB 30 r | 5 - 40 Gew.-% |
| Genapol X-080 r | 1 - 10 Gew.-% |
| Zitronensäure | 1 - 6 Gew.-% |
| Dipropylenglykol | 1 - 10 Gew.-% |
| Rhodopol T r | 0,1 - 0,5 Gew.-% |
| Parfümöl DROM r | 2 - 15 Gew.-% |
| Konservierungsmittel | q.s. |
| Farbe | q.s. |
| Wasser | ad 100 Gew.-% |

19. Zubereitung, insbesondere für WC-Erfrischer, umfassend:
| | |
|---|---|
| Lauryldimethylaminoessigsäurebetain | Lauryldimethylaminoessig- |
| | 5 - 30 Gew.-% |
| Isotridecylalkoholpolyglykolether 8 Mol/EO | 1 - 10 Gew.-% |
| Zitronensäure | 1 - 6 Gew.-% |
| Dipropylenglykol | 1 - 10 Gew.-% |
| Polyolfettsäureester | 0,5 - 6 Gew.-% |
| (solche gemäß Beschreibung) Parfümöl | 2 - 15 Gew.-% |
| Konservierungsmittel | q.s. |
| Farbe | q.s |
| Wasser | ad 100 Gew.-% |

20. Zubereitung, insbesondere für WC-Erfrischer, umfassend:
| | |
|---|---|
| Cocodimethylaminoxid | 5 - 30 Gew.-% |
| Isotridecylalkohlpolyglykolether 8 Mol/EO | 1 - 10 Gew.-% |
| Zitronensäure | 1 - 6 Gew.-% |
| Dipropylenglykol | 1 -10 Gew.-% |
| Polyolfettsäureester | 0,5 - 6 Gew.-% |
| (solche gemäß Beschreibung) Parfümöl | 2 - 15 Gew.-% |
| Konservierungsmittel | q.s. |
| Farbe | q.s. |
| Wasser | ad 100 Gew.-% |

21. Zubereitung, insbesondere für WC-Erfrischer, umfassend:
| | |
|---|---|
| Alkylamidopropylbetain (solche gemäß Beschreibung) | 1 - 50 Gew.-% |
| Fettaminpolyethylenglykolether C₆-C₂₂, 1-20 Mol EO | 1 - 10 Gew.-% |
| Polyolfettsäureester (solche gemäß Beschreibung) | 0 - 5 Gew.-% |
| Dinatriumlaurethsulfosuccinat | 0 - 5 Gew.-% |
| Parfümöl | 1 - 15 Gew.-% |
| Farbe | q.s. |
| Konservierungsmittel | q.s. |
| Zitronensäure | q.s. |
| Wasser | ad 100 Gew.-% |
